Europäisches Patentamt
European Patent Office
Office européen des brevets

(19)

(11) Veröffentlichungsnummer: **0 208 161 A1**

(12) **EUROPÄISCHE PATENTANMELDUNG**

(21) Anmeldenummer: 86108018.2

(51) Int. Cl.⁴: C07D 285/00

(22) Anmeldetag: 12.06.86

(30) Priorität: 15.06.85 DE 3521556

(43) Veröffentlichungstag der Anmeldung: 14.01.87 Patentblatt 87/03

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Rohr, Wolfgang, Dr.
In der Dreispitz 13
D-6706 Wachenheim(DE)
Erfinder: Seppelt, Wolfgang, Dr.
Lucas-Cranach-Strasse 8
D-6712 Bobenheim-Roxheim(DE)
Erfinder: Sproesser, Linhard, Dr.
Goethestrasse 4
D-6702 Bad Duerkheim(DE)

# (54) Verfahren zur Herstellung von 3,5-dialkylierten 1,2,3,5-Thiatriazolidin-4(2H)-on-1,1-dioxiden.

(57) Verfahren zur Herstellung von 3,5-disubstituierten 1,2,3,5-Thiatriazolidin-4(2H)-on-1,1-dioxiden der Formel I

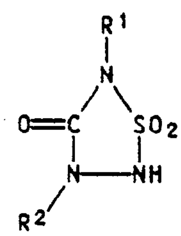

(I),

in der

$R^1$ unsubstituiertes oder durch Methoxy oder Halogen substituiertes Alkyl mit bis zu 10 Kohlenstoffatomen und

$R^2$ Alkyl mit bis zu 6 Kohlenstoffatomen oder Cycloalkyl mit 4 bis 8 Kohlenstoffatomen bedeuten,

durch Umsetzung eines entsprechenden 2-Halosulfonylcarbamoylhalogenids (II) mit dem entsprechenden Hydrazin (III) nach der Umsetzungsgleichung

EP 0 208 161 A1

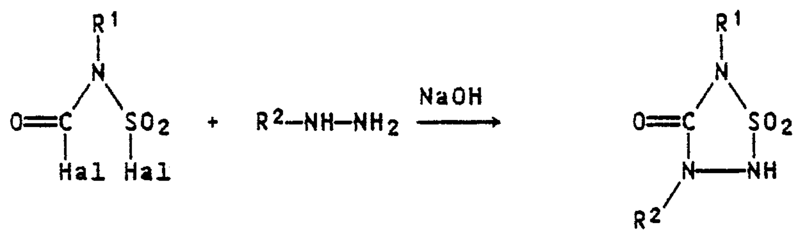


(II) (III) (I)

in Gegenwart von Alkalilauge, wobei man die Umsetzung in einem Zug in Gegenwart einer mindestens stöchiometrischen Menge an Alkalilauge einerseits und eines mit Wasser nicht unbeschränkt mischbaren organischen Lösungsmittels andererseits oberhalb von -50°C vornimmt.

# Verfahren zur Herstellung von 3,5-dialkylierten 1,2,3,5-Thiatriazolidin-4(2H)-on-1,1-dioxiden

Es ist bekannt, 3,5-Dialkyl-1,2,3,5-thiatriazolidin-4(2H)-on-1,1-dioxide bei -50°C durch Umsetzung von wasserfreiem Alkylhydrazin mit Alkylaminosulfonylhalogeniden herzustellen (J. Chem. Res. 1977, S. 2813-2825). Diese Verbindungen sind wertvolle Zwischenprodukte für die Herstellung von Insektiziden (EP-A-33 456).

Das beschriebene Herstellverfahren ist jedoch für eine Übertragung in den technischen Maßstab wenig geeignet, da es bei sehr tiefer Temperatur abläuft, und das Arbeiten mit wasserfreiem Alkylhydrazin (insbesondere mit Monomethylhydrazin) ist auch wegen der leichten Brennbarkeit, der Explosionsgefährlichkeit und schließlich wegen der bedenklichen toxikologischen Eigenschaften dieser Verbindungen bei technischer Verwendung mit hohem Aufwand verbunden.

Schließlich ist die Aufarbeitung der Umsetzungsprodukte, die z.B. eine Kugelrohrdestillation erfordert, wenig vorteilhaft.

Aufgabe der vorliegenden Erfindung ist es daher, ein Verfahren zur Herstellung von Verbindungen der Formel I

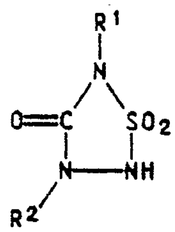
(I),

in der

$R^1$ unsubstituiertes oder durch Methoxy oder Halogen substituiertes Alkyl mit bis zu 10 Kohlenstoffatomen und

$R^2$ Alkyl mit bis zu 6 Kohlenstoffatomen oder Cycloalkyl mit 4 bis 8 Kohlenstoffatomen bedeuten,

durch Umsetzung eines entsprechenden 2-Halosulfonylcarbamoylhalogenids (II) mit dem entsprechenden Hydrazin (III) nach der Umsetzungsgleichung

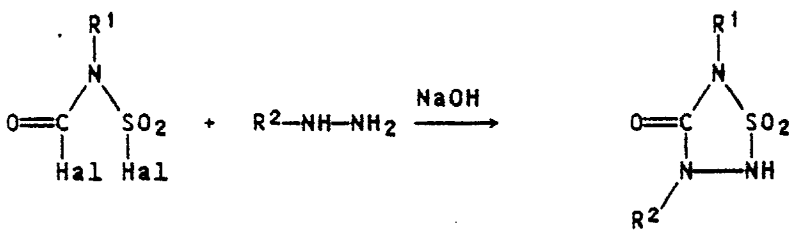


(II) (III) (I)

zu finden, das die bisherigen Nachteile vermeidet und unter technischen Bedingungen in guter Ausbeute und Reinheit die gesuchten Produkte liefert.

Die Erfindung bezieht sich insbesondere auf solche Thiatriazolidine (I), bei denen $R^1$ der Formel I für unverzweigte oder verzweigte Alkylreste mit bis zu 10 Kohlenstoffatomen, vorzugsweise bis zu 4 Kohlenstoffatomen steht, die durch Methoxy oder Halogen, wie Chlor substituiert sein können. Beispiele für derartige Reste sind Methyl, Ethyl, Isopropyl, 2-Chlorethyl, n-Butyl, sec.-Butyl, i-Butyl. $R^2$ in Formel I steht für unverzweigte oder verzweigte Alkylreste mit bis zu 4 Kohlenstoffatomen, wie Methyl, Ethyl, n-Propyl, i-Propyl, sec.-Butyl, i-Butyl, oder Cycloalkylreste mit 4 bis 8 Kohlenstoffatomen, wie Cyclobutyl, Cyclohexyl, Cyclooctyl.

Erfindungsgemäß gewinnt man die Thiatriazolidine (I) durch Umsetzung eines 2-substituierten 2-Halosulfonylcarbamoylhalogenids mit einem Alkylhydrazin bei Temperaturen oberhalb -50°C in Gegenwart von wäßriger Alkalilauge, indem man die Umsetzung in einem Zug in Gegenwart einer mindestens stöchiometrischen Menge an Alkalilauge und einem mit Wasser nicht unbeschränkt mischbaren organischen Lösungsmittel, d.h. zweiphasig durchführt. Man kann dabei auch so vorgehen, daß man zunächst aus einem Salz des Alkylhydrazins mit Alkalilauge im geschlossenen Gefäß das Alkylhydrazin freisetzt, die nunmehr noch erforderliche stöchiometrische Menge an Alkalilauge nach und nach oder auf einmal zugibt und zu diesem Gemisch, dem noch ein mit Wasser nicht mischbares Lösungsmittel zugesetzt wurde, das Carbamoylhalogenid nach und nach zufügt. Halogenid im Sinne der Erfindung bedeutet insbesondere Chlorid und evtl. auch Bromid und Fluorid, wobei in der Verbindung II die beiden Halogenatome gleich oder verschieden sein können.

Als mit Wasser nicht oder nicht unbegrenzt mischbares Lösungsmittel verwendet man nach fachmännischen Gesichtspunkten geeignete organische Verbindungen, die also bei der Reaktionstemperatur flüssig sind und mit den Reaktionsteilnehmern sich nicht umsetzen.

Geeignet sind z.B. aliphatische und aromatische Kohlenwasserstoffe und deren Halogenverbindungen als reine Stoffe oder in Form technischer Gemische. Sie müssen offensichtlich nicht besonders getrocknet sein oder sonstige spezielle Anforderungen erfüllen. Die in den Beispielen verwendeten, relativ großen Mengen können bei technischer Ausgestaltung ohne weiteres unterschritten werden.

Die Umsetzung verläuft schon bei niedriger Temperatur, z.B. ab -50°C bis +120°C, bevorzugt im Bereich von -10°C bis +50°C und insbesondere bei -10 bis +10°C. Unter diesen Bedingungen wird weder die Regioselektivität der Reaktion beeinflußt, noch findet eine merkliche Hydrolyse der bifunktionellen Säurehalogenide II in dem stark alkalischen Milieu statt, sondern es erhöht sich im Gegenteil bei dieser Reaktionsführung die Ausbeute der Reaktion merklich.

Besonders glatt verläuft die Umsetzung, wenn man ein Salz des Alkylhydrazins, beispielsweise das Sulfat vorlegt, das organische Lösungsmittel und gegebenenfalls Wasser zufügt, dann zuerst mit der stöchiometrischen Menge Alkalilauge im geschlossenen Gefäß das Methylhydrazin freisetzt, mindestens drei weitere Äquivalente Alkalilauge zuführt und dann das Carbamoylhalogenid zugibt. Bei dieser Verfahrensweise wird das entstehende Zielprodukt in das entsprechende Alkalisalz überführt, das in der wäßrigen Phase verbleibt. Die organische Phase kann entfernt und, falls gewünscht, können mit weiterem organischem Lösungsmittel eventuell vorhandene organische Verunreinigungen entfernt werden. Die dem Reaktionsgefäß entnommenen organischen Phasen füllt man direkt in Gefäße, in denen sich eine geringe Menge wäßriger Säure befindet, um eventuell noch vorhandene Restmengen Alkylhydrazin in das entsprechende Salz zu überführen. Nach Ansäuern des Reaktorinhalts wird der mit Wasser nicht mischbare Heterocyclus isoliert oder, gegebenenfalls nach Verdünnen mit weiterem organischem Lösungsmittel, mit Wasser extrahiert.

Die nachstehenden, im Labormaßstab angegebenen Beispiele lassen sich mit üblichen verfahrenstechnischen Überlegungen leicht in den technischen oder großtechnischen Maßstab übertragen.

Beispiel 1

Zu einer Mischung aus 138 g (3 mol) Methylhydrazin, 2,5 l Dichlormethan und 276 ml Wasser werden bei -10°C 660 g (3 mol) 2-Chlorsulfonyl-2-isopropylcarbamoylchlorid nach und nach zugesetzt, 1 Stunde bei -10°C nachgerührt, bei derselben Temperatur 360 g Natriumhydroxid, gelöst in 2,5 l Wasser, allmählich zugesetzt und 12 Stunden bei Raumtemperatur gerührt. Die Phasen werden getrennt, die organische Phase einmal mit wäßriger verd. Natronlauge gewaschen und das Waschwasser mit der wäßrigen Phase vereinigt. Diese wird nunmehr mit konz. Salzsäure angesäuert, dreimal mit 200 ml Dichlormethan extrahiert, dieses über Natriumsulfat getrocknet und eingedampft. Man gewinnt 427 g, entsprechend 73,7 % der, auf Methylhydrazin bezogen, berechneten Ausbeute.

Beispiel 2

43,2 g (0,3 mol) Methylhydrazinsulfat werden in 200 ml Dichlormethan suspendiert, 48 g (1,2 mol) Natriumhydroxid in 200 ml Wasser so zugesetzt, daß die Temperatur nicht über 30°C steigt. Dann werden 66 g (0,3 mol 2-Chlorsulfonyl-2-isopropylcarbamoylchlorid ebenfalls bei unter 30°C langsam zugesetzt, weitere 24 g (0,6 mol) Natriumhydroxid in 100 ml Wasser zugegeben, 12 Stunden gerührt, die Phasen getrennt, die wäßrige Phase mit konz. Salzsäure angesäuert, dreimal mit 250 ml Dichlormethan extrahiert, über Natriumsulfat getrocknet und eingedampft.

Ausbeute: 49 g entsprechend 84,6 % (berechnet wie vor).

Beispiel 3

Durchführung wie Beispiel 2, nur daß sofort die gesamte Menge an Natronlauge vor dem Carbamoylchlorid zugegeben wird.

Ausbeute: 48,7 g entsprechend 84,1 % (berechnet wie vor).

Die in der nachstehenden Tabelle -zusammen mit den nach Beispiel 1 bis 3 erhaltenen Verbindungen -aufgeführten Stoffe wurden hergestellt, soweit sie durch physikalische Eigenschaften gekennzeichnet sind; die nicht gekennzeichneten können aus den entsprechenden Rohstoffen leicht unter Abwandlung der experimentellen Angaben der Beispiele 1 bis 3 erhalten werden.

Tabelle

| Beispiel Nr. | $R^1$ | $R^2$ | $n_D^{25}$ | Fpt. |
|---|---|---|---|---|
| 1 | i-$C_3H_7$ | $CH_3$ | | |
| 2 | i-$C_3H_7$ | $CH_3$ | 1.4730 | 180°C |
| 3 | i-$C_3H_7$ | $CH_3$ | | |
| 4 | $C_2H_5$ | $CH_3$ | 1.4780 | |
| 5 | $CH_3$ | $CH_3$ | | 60-62°C |
| 6 | $CH_3$ | i-$C_3H_7$ | | 68-71°C |
| 7 | i-C§H7 | i-$C_3H_7$ | | 58-60°C |
| 8 | i-$C_3H_7$ | cyclo-$C_6H_{11}$ | | 95-98°C |

**Ansprüche**

1. Verfahren zur Herstellung von 3,5-disubstituierten 1,2,3,5-Thiatriazolidin-4(2H)-on-1,1-dioxiden der Formel I

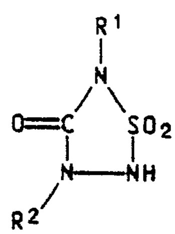

(I),

in der

$R^1$ unsubstituiertes oder durch Methoxy oder Halogen substituiertes Alkyl mit bis zu 10 Kohlenstoffatomen und

$R^2$ Alkyl mit bis zu 6 Kohlenstoffatomen oder Cycloalkyl mit 4 bis 8 Kohlenstoffatomen bedeuten,

durch Umsetzung eines entsprechenden 2-Halosulfonylcarbamoylhalogenids (II) mit dem entsprechenden Hydrazin (III) nach der Umsetzungsgleichung

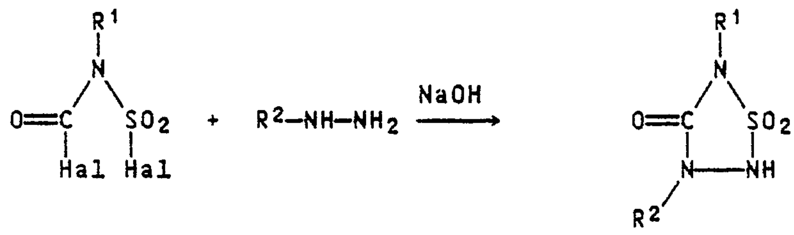



(II) (III) (I)

in Gegenwart von Alkalilauge, dadurch gekennzeichnet, daß man die Umsetzung in einem Zug in Gegenwart einer mindestens stöchiometrischen Menge an Alkalilauge einerseits und eines mit Wasser nicht unbeschränkt mischbaren organischen Lösungsmittels andererseits oberhalb von - 50°C vornimmt.

2. Verfahren nach Anspruch 1, dadurch gekennzeichnet, daß man, ausgehend von einem Salz des Alkylhydrazins, zunächst das Alkylhydrazin mit mindestens der äquivalenten Menge Lauge freisetzt, gegebenenfalls weitere bzw. die gesamte Menge Lauge hinzufügt und zu dem Gemisch das Carbamoylhalogenid nach und nach zusetzt.

3. Verfahren nach Anspruch 1, dadurch gekennzeichnet, daß man, ausgehend vom freien Alkylhydrazin, dem organischen Lösungsmittel und wäßrigen Alkali, ein Reaktionsgemisch herstellt und diesem das Carbamoylhalogenid zusetzt.

Europäisches Patentamt

# EUROPÄISCHER RECHERCHENBERICHT

Nummer der Anmeldung

EP 86 10 8018

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | CHEMICAL ABSTRACTS, Band 88, Nr.3, 16. Januar 1978, Seite 631, Zusammenfassung 22842v, Columbus, Ohio, US; D. BARTHOLOMEW et al.: "Heterocyclic synthesis using 2-alkyl-2-chloro- and -fluoro-sulfonylcarbamoyl chlorides" & J. CHEM. RES. (S) 1977, (10), 238<br>----- | 1-3 | C 07 D 285/00 |

RECHERCHIERTE SACHGEBIETE (Int. Cl.4)

C 07 D 285/00

Der vorliegende Recherchenbericht wurde für alle Patentansprüche erstellt.

| Recherchenort | Abschlußdatum der Recherche | Prüfer |
|---|---|---|
| DEN HAAG | 22-09-1986 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82